# FASCICULE DE BREVET EUROPEEN

(11) **EP 3 405 254 B1**
(45) Date de publication et mention de la délivrance du brevet: **04.12.2019**
(21) Numéro de dépôt: 17703325.5
(22) Date de dépôt: 09.01.2017
(51) Int. Cl.: A61N 1/05, A61N 1/36

(54) **DISPOSITIF MÉDICAL IMPLANTABLE ACTIF DE NEUROSTIMULATION À ÉLECTRODES SECTORIELLES COMMUTABLES SELON DES CONFIGURATIONS DIFFÉRENTES**
AKTIVE IMPLANTIERBARE MEDIZINISCHE NEUROSTIMULATIONSVORRICHTUNG MIT JE NACH VERSCHIEDENEN KONFIGURATIONEN SCHALTBAREN SEKTORELLEN ELEKTRODEN
ACTIVE IMPLANTABLE MEDICAL NEUROSTIMULATION DEVICE WITH SECTORAL ELECTRODES THAT ARE SWITCHABLE ACCORDING TO VARIOUS CONFIGURATIONS

(30) Priorité: 20.01.2016 FR 1650446
(43) Date de publication de la demande: 28.11.2018
(73) Titulaire: Sorin CRM SAS, 92140 Clamart (FR)
(72) Inventeur: CHATALIC, Guillaume, 92140 Clamart (FR); LAFLUTTE, Marc, 78000 Versailles (FR); BIERG, Jean-Claude, 93470 Coubron (FR)
(74) Mandataire: Grünecker Patent- und Rechtsanwälte PartG mbB
(86) Numéro de dépôt international: PCT/EP2017/050349
(87) Numéro de publication internationale: WO 2017/125274

(56) Documents cités:
- US-A1- 2010 106 219
- US-A1- 2011 106 215
- US-A1- 2011 125 224
- US-A1- 2011 160 810
- US-A1- 2014 100 632

## Description

L'invention concerne les "dispositifs médicaux implantables actifs" tels que définis par la Directive 90/385/CEE du 20 juin 1990 du Conseil des communautés européennes.

Elle concerne plus précisément les implants permettant de délivrer des thérapies de stimulation électrique consistant à appliquer sur des organes, à des fins thérapeutiques, une stimulation sous forme d'impulsions électriques répétées.

En particulier, la stimulation électrique du système nerveux est une approche thérapeutique reconnue ou en cours d'évaluation à l'égard de très nombreux troubles tels que l'épilepsie, la douleur, l'insuffisance cardiaque, l'apnée du sommeil, l'obésité, etc.

La thérapie peut être délivrée de diverses manières - toutes incluses dans le champ de la présente invention - au moyen d'une sonde de neurostimulation disposée autour, à proximité ou à l'intérieur de la structure organique ciblée.

L'invention concerne plus spécifiquement les dispositifs implantables permettant de délivrer une telle thérapie de stimulation du système nerveux (ci-après désignée de façon générale "neurostimulation"), notamment mais de façon non limitative par une stimulation du nerf vague, technique dite "VNS" (*Vagus Nerve Stimulation*)*.*

Le dispositif comprend un générateur délivrant des impulsions électriques de courant, associé à une sonde de neurostimulation constituée dans le cas le plus courant d'un manchon enroulé autour du nerf. Ce manchon est pourvu d'une pluralité d'électrodes venant s'appliquer contre le nerf de façon à en stimuler sélectivement certaines régions par une répartition contrôlée des courants appliqués aux diverses électrodes. Il s'agit notamment, grâce à une telle répartition, de cibler l'activation de certaines fibres ou axones disposés dans la région visée.

Dans la suite de la description, on fera principalement référence à ce mode de délivrance de la thérapie de neurostimulation, mais on comprendra qu'il ne présente pas de caractère limitatif, l'invention s'appliquant aussi bien à d'autres types de sondes, notamment les sondes tubulaires en forme de stent introduites à l'intérieur d'un vaisseau, par exemple l'aorte, pour stimuler certains sites barorécepteurs ayant un effet indirect sur le système nerveux, ou encore les sondes implantées directement à l'intérieur de l'organe, typiquement un nerf ou le cerveau, pour une stimulation directe, *in situ*, du système nerveux.

Si l'on prend le cas de la stimulation du nerf vague, celui-ci comprend de nombreux axones qui innervent divers organes et muscles du corps humain. Certains de ces axones innervent l'organe destiné à faire l'objet de la thérapie (organe cible), tandis que d'autres innervent des organes qui ne sont pas concernés par la thérapie.

Ainsi, une stimulation globale, non différenciée, d'un nerf peut, au-delà de l'effet thérapeutique désiré, induire des effets indésirables au niveau d'autres organes ou retours sensoriels. Au surplus, pour avoir l'effet thérapeutique souhaité via le ou les axones concernés, une excitation non différenciée de l'ensemble des fibres d'un nerf peut nécessiter un courant électrique bien plus élevé que ce qui est nécessaire pour le seul effet thérapeutique recherché.

Il importe donc de délivrer une stimulation spatialement sélective de l'organe cible (typiquement, mais de façon non limitative, un nerf tel que le nerf vague), afin d'aboutir à un effet focalisé sur les paramètres physiologiques visés, en limitant les effets indésirables sur des organes ou muscles non visés et en limitant le courant électrique nécessaire pour la stimulation.

Le EP 2 946 806 A1 (Sorin CRM) décrit un dispositif de neurostimulation comportant une sonde de stimulation munie à cet effet d'électrodes agencées selon plusieurs zones annulaires réparties dans la direction longitudinale du nerf vague et portées par un manchon entourant celui-ci. Chaque zone annulaire regroupe une pluralité d'électrodes sectorielles, c'est-à-dire d'électrodes occupant des secteurs angulaires distincts circonférentiellement répartis sur la zone annulaire.

Les électrodes sectorielles de certaines zones annulaires peuvent être toutes électriquement reliées ensemble pour se comporter, vis-à-vis de la stimulation, de façon analogue à une électrode annulaire formée d'un seul tenant.

L'invention concerne le cas où, au contraire, les électrodes sectorielles sont des électrodes dites "de sélectivité", c'est-à-dire connectées individuellement et distinctement au générateur d'impulsions de façon que chacune d'entre elles puisse jouer soit le rôle d'une anode (point d'entrée du courant dans le nerf), soit le rôle d'une cathode (point de sortie du courant dans le nerf), soit être électriquement inactive (comportement en haute impédance).

Dans un exemple typique, ces électrodes sectorielles sont au nombre de quatre dans chaque zone annulaire, et sont espacées de 90° l'une par rapport à la suivante. Par convention, pour les distinguer, ces quatre électrodes seront désignées "Ouest", "Nord", "Est" et Sud" (O, N, E et S), les électrodes Nord et Sud, ou Ouest et Est, étant disposées en vis-à-vis à 180° l'une de l'autre tandis que les électrodes Ouest et Nord, ou Nord et Est, etc. sont disposées adjacentes à 90° l'une de l'autre.

Ce nombre de quatre électrodes sectorielles n'est toutefois pas limitatif, les configurations comprenant par exemple deux ou huit électrodes, régulièrement espacées ou non, étant également envisageables.

Il est ainsi possible de définir au niveau d'une même zone annulaire une pluralité de configurations polaires de stimulation : par exemple, dans le cas de quatre électrodes, l'une des électrodes est connectée en tant que cathode, ses deux électrodes voisines sont connectées en tant qu'anodes, et l'électrode opposée est non connectée. Cette configuration peut être transposée par rotations successives de 90°, donnant ainsi quatre configurations de stimulation différentes possibles. Bien entendu, toute autre combinaison de connexion peut être également testée.

De façon générale, par "configuration de stimulation", on entendra toute configuration combinant les critères suivants :
i) le fait qu'une électrode soit ou non une électrode active (c'est-à-dire parcourue ou non par un courant de stimulation) ;
ii) la polarité, anode ou cathode, de chaque électrode active (c'est-à-dire le sens du courant traversant cette électrode) ; et
iii) la répartition entre les différentes électrodes actives du courant produit par le générateur de neurostimulation (utilisation de plusieurs anodes et/ou plusieurs cathodes).

Du côté du générateur, la structure conventionnelle consiste à prévoir une pluralité de sources de courant et une pluralité de puits de courant.

Les sources de courant sont des générateurs de courant permettant d'injecter le courant dans le nerf au niveau d'une électrode formant anode, tandis que les puits de courant sont des générateurs de courant qui extraient du nerf, par une électrode formant cathode, le courant qui y avait été injecté par la ou les sources. Un couple source/puits est dédié à chacune des électrodes, de sorte que les sources et les puits sont en nombre égal à celui des électrodes (par exemple quatre sources et quatre puits pour une configuration à quatre électrodes sectorielles Ouest-Nord-Est-Sud). Chacune des sources et chacun des puits est associé de manière statique et univoque à une électrode particulière pour laquelle la source ou bien le puits qui lui est associé peut être sélectivement activé : si la source est activée, l'électrode jouera le rôle d'une anode, si le puits est activé, l'électrode jouera le rôle d'une cathode, et si ni la source ni le puits ne sont activés, l'électrode sera inactive.

Le US 2003/0139781 A1 décrit une telle structure de générateur, avec des sources et des puits de courant sélectivement commutables à des électrodes respectives, cette structure étant décrite dans le cadre d'une sonde multi-électrodes de stimulation de la moelle épinière (SCS, *Spinal Chord Stimulation*).

En jouant sur les commutations des sources et des puits de courant aux différentes électrodes qui leur sont associées, il est possible de tester plusieurs configurations de stimulation prédéterminées, notamment pour rechercher une configuration de stimulation optimale qui :
- maximise l'effet physiologique produit par la thérapie de neurostimulation, et
- minimise les effets secondaires indésirables induits du fait de la stimulation du nerf (par exemple le déclenchement d'une toux).

Le EP 2 946 806 A1 précité décrit une technique permettant de rechercher de façon entièrement automatique une telle configuration de stimulation optimale en fonction de l'effet physiologique recherché.

Le test des différentes configurations de stimulation présuppose toutefois, pour que les effets physiologiques produits soient comparables, que d'une configuration à l'autre i) le courant total traversant le nerf soit le même et que ii) si plusieurs électrodes sont utilisées en tant qu'anodes ou en tant que cathodes, l'injection des courants au niveau de chaque cathode et de chaque anode soit fidèle à une consigne donnée, qui n'est pas nécessairement équilibrée.

Mais concrètement, tel n'est pas le cas. En effet, le test des différentes configurations de stimulation conduit à choisir d'utiliser des sources de courant différentes du générateur, et ces sources de courant ne délivrent pas des courants identiques.

En effet, les sources de courant (et les puits de courant également) sont des structures microélectroniques analogiques sujettes à des imprécisions lors de leur processus de fabrication : en pratique, aucun processus de fabrication ne permet d'assurer une géométrie strictement identique au niveau de l'ensemble des transistors à la base des circuits microélectroniques, les oxydations, dopages, lithographies, etc., n'étant jamais parfaitement identiques d'un transistor à l'autre. Ce phénomène dit de *mismatch*, propre à la nature analogique des sources de courant à base de circuits microélectroniques, se traduit par le fait que, pour une consigne de courant donnée constante, on observe des courants différents générés par les sources, et donc des courants différents sur les pôles de stimulation de l'organe selon la configuration polaire utilisée, du fait que d'une configuration à l'autre ce ne sont pas les mêmes sources de courant qui sont utilisées.

Ce phénomène conduit à l'apparition d'un biais sur le courant total réellement délivré au nerf, courant qui diffèrera du courant de consigne servant de référence pour l'évaluation de l'effet physiologique produit et la comparaison des effets produits entre des configurations de stimulation différentes.

L'introduction de tels biais peut alors conduire à de mauvaises interprétations cliniques des effets physiologiques de la neurostimulation : en particulier, en présence de ce type de biais, il devient impossible de discriminer les variations d'un paramètre physiologique qui sont réellement dues à la sélection d'une configuration polaire de stimulation différente, de celles introduites par les *mismatches.*

Un autre biais, outre celui de la différence entre le courant moyen réellement délivré au nerf et le courant de consigne, est celui du caractère non constant de la répartition du courant au niveau des électrodes d'une configuration polaire à l'autre. Par exemple, dans le cas de l'utilisation de deux anodes à consigne équivalente, il ne sera pas possible d'obtenir une répartition à 50/50 des courants entre ces deux électrodes. Ce biais supplémentaire pourra également engendrer des erreurs d'interprétation sur l'efficacité de la configuration de stimulation testée.

Le US2011/0125224 décrit un système de neuromodulation comprenant une pluralité d'électrodes utilisant une pluralité de sources qui peuvent être reconfigurées pour basculer d'une seconde polarité à une première polarité.

Le but de l'invention est de proposer une nouvelle architecture de générateur garantissant que pour une même consigne d'entrée, malgré les inévitables *mismatches* entre générateurs de courant et entre sources et puits, le courant injecté dans l'organe cible sera le même quelle que soit la configuration de stimulation utilisée, et que la répartition du courant sera constante quelque soit la configuration de stimulation utilisée.

L'idée de base de l'invention consiste à s'affranchir de l'architecture classique où une source de courant et un puits de courant sont dédiés à chaque pôle de stimulation (chaque électrode), selon une relation biunivoque. L'invention propose, à l'opposé, de dissocier les sources de courant des pôles de stimulation et de permettre une attribution dynamique de chaque source de courant du générateur à n'importe quel pôle de stimulation, et de même pour les puits de courant.

Il devient ainsi en particulier possible de commuter plusieurs sources de courant conjointement à un même pôle, avec pour résultat de moyenner le courant injecté par les différentes sources utilisées : le courant traversant l'organe cible sera alors globalement constant quelle que soit la configuration de stimulation sélectionnée.

Un autre avantage de cette architecture est de pouvoir utiliser des sources et des puits de courant en nombre supérieur (ou inférieur) à celui des électrodes - alors que dans une architecture classique, du fait du caractère dédié des sources et des puits, ce nombre doit être identique à celui des électrodes.

Ainsi, en augmentant le nombre de sources de courant conjointement utilisées pour délivrer un courant à une électrode donnée, on pourra d'autant mieux lisser les *mismatches* entre ces différentes sources par effet de moyenne.

Plus précisément, l'invention propose un dispositif implantable de neurostimulation par injection contrôlée de courants électriques simultanément en plusieurs points d'un tissu physiologique comprenant, de manière en elle-même connue :
- une sonde de neurostimulation apte à être placée autour, à proximité ou à l'intérieur d'un organe, comportant au moins un agencement d'électrodes sectorielles aptes à former des pôles de stimulation avec passage d'un courant de neurostimulation entre au moins une anode et au moins une cathode d'une configuration de stimulation prédéterminée ; et
- un générateur d'impulsions électriques de courant, comprenant :
   - une pluralité de sources de courant ;
   - une pluralité de puits de courant ;
   - une première structure de distribution des courants issus desdites sources de courant, pour le couplage sélectif d'au moins l'une des sources de courant à une électrode, de sorte que cette électrode constitue une électrode active d'anode de ladite configuration de stimulation prédéterminée ;
   - une seconde structure de distribution des courants issus desdits puits de courant pour le couplage sélectif d'au moins l'un des puits de courant à une autre électrode, de sorte que cette autre électrode constitue une électrode active de cathode de ladite configuration de stimulation prédéterminée, L'agencement d'électrodes comprend M électrodes et le générateur comprend N sources de courant et N puits de courant, avec N = M ou N ≠ M, les N sources de courant et les N puits de courant sont définis indépendamment des M électrodes :
      - la première structure de distribution est apte à opérer un couplage indifféremment d'au moins l'une des N sources de courant à l'une quelconque des M électrodes ;
      - la seconde structure de distribution est apte à opérer un couplage indifféremment d'au moins l'un des N puits de courant à l'une quelconque des M électrodes ; et
      - le générateur comprend en outre des moyens de pilotage de la première et de la seconde structure de distribution, aptes à définir une combinaison de couplages d'une pluralité de sources de courant et/ou d'une pluralité de puits de courant procurant un même courant moyen de neurostimulation pour des configurations de stimulation prédéterminées respectives différentes. En outre les moyens de pilotage sont des moyens de pilotage multiphasique, aptes à produire cycliquement une pluralité de combinaisons de couplages pour une même configuration de stimulation, de manière à moyenner le courant de neurostimulation délivré par la pluralité de sources de courant et la pluralité de puits de courant.

Selon diverses caractéristiques subsidiaires avantageuses :
- ladite combinaison de couplages inclut un couplage de toutes les sources de courant, avec notamment une partie des N sources de courant couplée conjointement sur une première électrode d'anode, et la partie restante des N sources de courant couplée conjointement sur une seconde électrode d'anode, différente de la première électrode d'anode ;
- ladite combinaison de couplages inclut un couplage de tous les puits de courant, avec notamment une partie des N puits de courant couplée conjointement sur une première électrode de cathode, et la partie restante des N puits de courant couplée conjointement sur une seconde électrode de cathode, différente de la première électrode de cathode, ou bien avec les N puits de courant couplés conjointement sur une unique électrode de cathode.
De façon avantageuse :
- les moyens de pilotage sont aptes à définir une combinaison de couplages de chacune des sources de courant et de chacun des puits de courant successivement sur chacune des électrodes de la configuration de stimulation prédéterminée ;
- ladite pluralité de combinaisons de couplages produites cycliquement pour une même configuration de stimulation comprennent une permutation circulaire des N sources de courant ;
- ladite pluralité de combinaisons de couplages produites cycliquement pour une même configuration de stimulation comprennent une permutation aléatoire des N sources de courant ;
- les moyens de pilotage sont des moyens aptes à produire cycliquement ladite pluralité de combinaisons de couplages au cours de phases successives de durées respectives égales.

On va maintenant décrire un exemple de mise en œuvre de la présente invention, en référence aux dessins annexés où les mêmes références désignent d'une figure à l'autre des éléments identiques ou fonctionnellement semblables.
La Figure 1 est une vue d'ensemble d'un dispositif de neurostimulation implantable comprenant un générateur et une sonde de stimulation du nerf vague.
La Figure 2 est une représentation schématique d'une architecture de générateur conventionnel, avec des sources et des puits de courant dédiés, associés à un ensemble d'électrodes sectorielles respectives.
Les Figures 3A à 3D illustrent respectivement la manière d'obtenir, avec une architecture de générateur selon la Figure 2, quatre configurations polaires différentes de stimulation, avec indication des courants circulant effectivement dans les différents pôles de l'agencement d'électrodes sectorielles.
La Figure 4 est une représentation schématique d'une architecture de générateur selon l'invention, avec des sources et des puits de courant pouvant être affectés de manière dynamique et indifférenciée à n'importe lequel des pôles de stimulation.
Les Figures 5A à 5D illustrent respectivement la manière d'obtenir, avec une architecture de générateur selon l'invention, quatre configurations polaires différentes de stimulation, avec indication des courants circulant dans les différents pôles de l'agencement d'électrodes sectorielles.
La Figure 6 est un tableau comparatif donnant, pour une architecture classique et pour une architecture selon l'invention, les courants entrants ou sortants sur les différentes électrodes actives, en fonction de la configuration de stimulation choisie.
Les Figures 7A à 7D illustrent un perfectionnement de l'invention, avec production cyclique d'une pluralité de combinaisons de commutation pour une même configuration de stimulation.
La Figure 8 est un chronogramme représentant la succession des différentes phases de la technique illustrée Figures 7A à 7D.

On va maintenant décrire un exemple de réalisation du dispositif de l'invention, dans le cadre (non limitatif) d'un stimulateur VNS, c'est-à-dire de neurostimulation du nerf vague.

Un tel stimulateur comprend un microprocesseur programmable pourvu de circuits pour mettre en forme et délivrer des impulsions de stimulation à des électrodes implantées. Il est possible d'y transmettre par télémétrie des logiciels qui seront conservés en mémoire et exécutés pour mettre en œuvre les fonctions de l'invention qui seront décrites ci-dessous. L'adaptation de ces appareils à la mise en œuvre des fonctions de l'invention est à la portée de l'homme du métier, et elle ne sera pas décrite en détail. Des moyens logiciels participent à la mise en œuvre de l'invention, avec des algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs correspondant en pratique à une pluralité de fonctions globalement exécutées par un même logiciel.

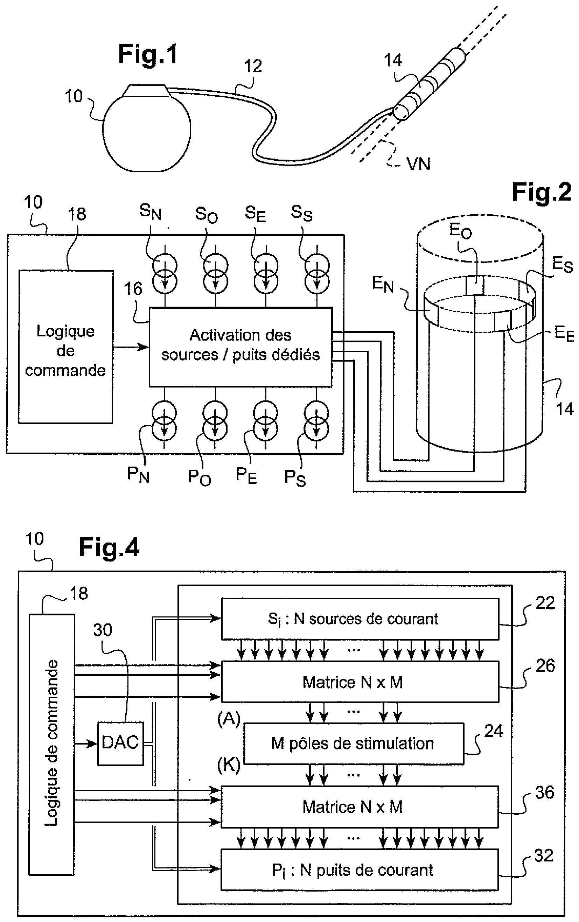

Sur la Figure 1, la référence 10 désigne le boitier d'un générateur implantable de stimulation du nerf vague (VNS). Cette stimulation est délivrée par une sonde 12 portant à sa partie distale un manchon 14 portant un agencement d'électrodes implantées autour du nerf vague VN, de manière à stimuler sélectivement certaines fibres de celui-ci en injectant des charges électriques produites par le générateur 10 sur certaines électrodes, comme on le verra en détail dans la suite.

La Figure 2 est un schéma synoptique d'une architecture conventionnelle de générateur 10 relié à un manchon 14 de sonde.

Sur la Figure 2, on a illustré quatre électrodes sectorielles E_{O}, E_{N}, E_{E} et E_{S}, disposées selon un agencement annulaire, chaque électrode occupant un secteur angulaire discret et étant espacée angulairement de 90° par rapport à la suivante. Par convention, ces électrodes correspondant chacune à un pôle de stimulation respectivement désigné "Ouest", "Nord", "Est" et "Sud", mais on comprendra que cet agencement n'est nullement limitatif et que l'on peut prévoir typiquement entre deux et huit électrodes, régulièrement espacées ou non.

La sonde 14 peut comprendre plusieurs agencements annulaires tels que celui illustré Figure 2, régulièrement répartis dans la direction longitudinale du nerf vague et portés par le même manchon. Les électrodes peuvent être reliées ensemble, pour se comporter de façon analogue à une électrode annulaire d'un seul tenant ou - comme dans l'exemple illustré correspondant au cas concerné par l'invention - constituées d'"électrodes de sélectivité" connectées individuellement au générateur d'impulsions de façon que chacune d'entre ces électrodes Ouest, Nord, Est et Sud puisse jouer soit le rôle d'une anode (A), soit le rôle d'une cathode (K), soit être non connectée (comportement en haute impédance).

Le générateur 10 auquel sont connectées ces électrodes comprend quatre générateurs de courant formant sources S_{N}, S_{O}, S_{E} et S_{S}, en nombre égal à celui des électrodes E_{N}, E_{O}, E_{E} et E_{S} respectives, auxquelles elles peuvent être sélectivement couplées par un circuit 16 piloté par une logique de commande numérique 18 activant individuellement chacune des sources de courant dédiées.

De la même façon, le générateur 10 comprend quatre puits de courant P_{N}, P_{O}, P_{E} et P_{S}, en nombre égal à celui des électrodes et sélectivement couplés à celles-ci par l'intermédiaire du circuit 16 piloté par la logique de commande numérique 18 activant individuellement chacun des puits de courant dédiés.

Ainsi, selon l'état d'activation du circuit 16, tel que défini par la logique de commande 18, chaque électrode Eᵢ sera soit couplée à sa source respective associée Sᵢ pour jouer le rôle d'une anode, soit à son puits de courant associé Pᵢ, pour jouer le rôle d'une cathode, soit être non connectée, l'électrode correspondante étant alors inactive.

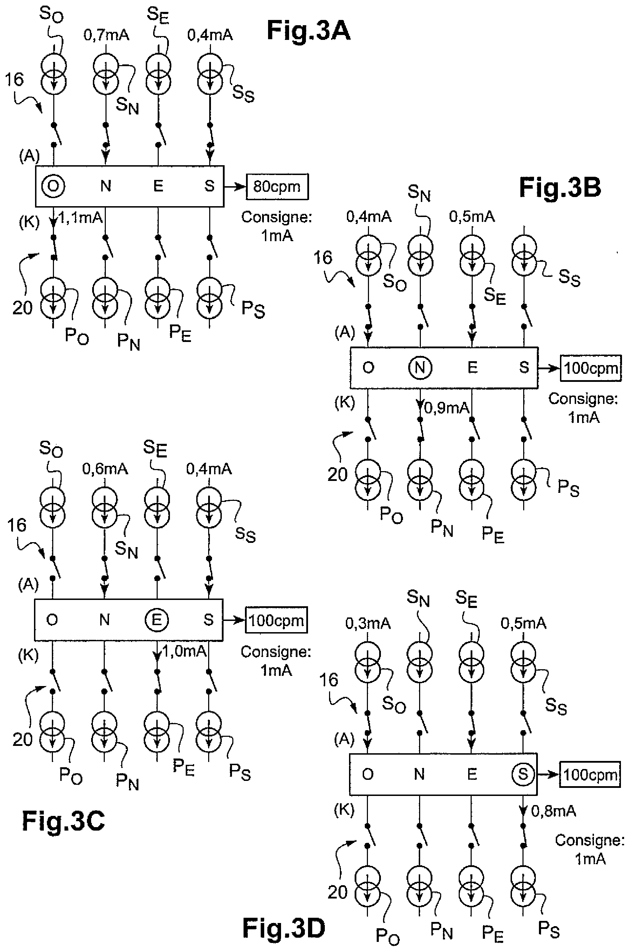

Les Figures 3A à 3D illustrent respectivement la manière d'obtenir, avec une telle architecture conventionnelle de générateur, quatre configurations polaires différentes de stimulation.

Dans l'exemple illustré par ces figures, on définit quatre configurations où :
- l'une des électrodes est connectée en tant que cathode (par exemple l'électrode Ouest sur la Figure 3A), avec une consigne en courant à 1 mA,
- l'électrode diamétralement opposée est non connectée (l'électrode Est sur la Figure 3A), et
- les deux électrodes voisines de l'électrode de cathode (les électrodes Sud et Nord sur la Figure 3A) sont connectées en tant qu'anodes, avec des consignes en courant à 0,5 mA.

La connexion en tant que cathode est opérée par connexion de l'électrode au puits de courant correspondant (l'électrode Ouest au puits P_{O} sur la Figure 3A) et la connexion en tant qu'anode aux sources de courant correspondantes (les électrodes Sud et Nord aux sources S_{S} et S_{N} sur la Figure 3A).

La configuration de stimulation obtenue correspondant à la configuration de la Figure 3A sera désignée "configuration de sélectivité Ouest".

La configuration de la Figure 3B est une "configuration de sélectivité Nord" obtenue par une rotation de 90° par rapport à la configuration de la

Figure 3A. De la même façon, la configuration de la Figure 3C est une "configuration de sélectivité Est" obtenue par une rotation de 90° par rapport à la configuration de la Figure 3B et la configuration de la Figure 3D est une "configuration de sélectivité Sud" obtenue par une rotation de 90° par rapport à la configuration de la Figure 3C.

On dispose ainsi dans cet exemple de quatre configurations possibles, tournées de 90°, qui permettent de stimuler sélectivement différentes fibres du nerf et produire ainsi des effets physiologiques différents.

On comprendra que d'autres configurations d'électrodes peuvent être également testées, par exemple des configurations avec une cathode et une anode adjacentes, une cathode et une anode opposées, une cathode et trois anodes, etc., ce qui permet de réaliser de multiples combinaisons permettant à chaque fois de stimuler de façon préférentielle une zone de la section du nerf ayant un certain contour.

Par ailleurs, il est possible non seulement de jouer sur la position des anodes pour focaliser différemment les courants, mais également de mettre en œuvre plusieurs cathodes, typiquement deux cathodes, pour pouvoir déplacer la zone annulaire et viser ainsi des fibres qui ne seraient pas localisées directement sous une cathode mais plutôt entre deux cathodes.

On comprendra également qu'en multipliant le nombre d'électrodes sectorielles, typiquement jusqu'à huit au lieu de quatre, on peut définir avec une meilleure résolution la région du nerf stimulé de façon privilégiée.

Les comparaisons que l'on peut effectuer entre les différentes configurations polaires de stimulation supposent bien évidemment que les courants injectés dans le nerf présentent la même intensité et soient répartis de la même façon d'une configuration à l'autre.

Ainsi, dans l'exemple des Figures 3A à 3D, on supposera que le courant total injecté dans le nerf, correspondant au courant recueilli côté cathode, est un courant de consigne de 1 mA, résultant de l'injection de 0,5 + 0,5 mA par les sources de courant côté anodes.

Concrètement, tel n'est pas le cas, en raison du *mismatch* expliqué plus haut résultant des aléas de fabrication des structures microélectroniques, d'une source à l'autre et entre source et puits associés. Ce phénomène introduit deux biais :
- en valeur absolue, on constate que le courant total traversant le nerf n'est pas nécessairement égal au courant de consigne, et peut varier dans des proportions importantes d'une configuration à l'autre : dans l'exemple des Figures 3A à 3D, ce courant est de 1,1 mA pour la configuration de sélectivité Ouest, 0,9 mA pour la configuration de sélectivité Nord, 1,0 mA pour la configuration de sélectivité Est et de 0,8 mA pour la configuration de sélectivité Sud ;
- d'autre part, la consigne au niveau des anodes (0,5 mA pour E_{N} et 0,5 mA pour E_{S} dans le cas de la Figure 3A) n'étant pas exactement respectée, l'injection de courant est déséquilibrée : par exemple dans la configuration de sélectivité Ouest on observe des courants de 0,7 mA/0,4 mA, soit 64/36, dans la configuration de sélectivité Nord de 0,4 mA/0,5 mA, dans la configuration de sélectivité Est 0,6 mA/0,4 mA et dans la configuration de sélectivité Sud 0,3 mA/0,5 mA.

Ces biais modifient de façon significative, et d'une manière qui n'est ni souhaitée ni contrôlable, l'effet physiologique obtenu, ce qui fausse la recherche d'une configuration polaire de stimulation optimale.

La recherche d'une telle configuration optimale consiste à évaluer, pour chaque configuration possible, l'effet produit sur un paramètre physiologique donné : fréquence cardiaque, contractilité cardiaque, électroneurogramme, etc.

Si l'on prend l'exemple de la fréquence cardiaque, on sait que l'application d'une neurostimulation a pour effet de réduire cette fréquence, par modification de l'équilibre du système sympathovagal (effet chronotrope négatif de la neurostimulation).

Dans les exemples des Figures 3A à 3D on constate que pour la configuration de sélectivité Ouest (Figure 3A) la fréquence cardiaque est de 80 cpm, tandis que pour chacune des autres configurations de sélectivité Nord, Est et Sud (Figures 3B, 3C et 3D), cette fréquence est de 100 cpm. De fait, en première analyse la configuration de sélectivité Ouest semblerait la meilleure, les configurations de sélectivité Nord, Est et Sud ne produisant pas d'effet chronotrope.

Mais ce résultat étant affecté du biais expliqué plus haut, il n'est pas possible de déterminer si la fréquence cardiaque moindre observée :
- est un effet de la sélectivité (choix d'une configuration polaire particulière) ;
- ou un effet du *mismatch* : en effet, dans la configuration de la Figure 3A, le courant injecté (1,1 mA) est supérieur à celui injecté dans les autres configurations (respectivement 0,9, 1,0 et 0,8 mA). L'effet chronotrope négatif observé avec la configuration de la Figure 3A peut ainsi provenir, partiellement ou totalement, de l'injection d'un courant de neurostimulation plus élevé.

Tel est le problème que l'invention cherche à résoudre.

Pour cela, l'invention propose une architecture de générateur et un pilotage des sources/puits de courant différents de ce que l'on vient de décrire en référence aux Figures 2 et 3A à 3D.

La Figure 4 illustre de façon schématique l'architecture du générateur 10 selon l'invention.

Côté anode (partie supérieure de la Figure 4), le générateur 10 comprend un ensemble 22 de N sources de courant Sᵢ aptes à être couplées à un ensemble 24 de M pôles de stimulation (M électrodes indépendantes) par l'intermédiaire d'une matrice de commutation N x M 26 pilotée par la logique de commande numérique 18 via des liaisons numériques 28. La logique de commande pilote également un convertisseur numérique/analogique 30 pour le contrôle des sources de courant Sᵢ.

De façon similaire, côté cathode (partie inférieure de la Figure 4), le générateur 10 comprend un ensemble 32 de N puits de courant Pᵢ reliés aux M pôles de stimulation 24 par une matrice de commutation N x M 36 commandée par la logique de commande 18 via des liaisons numériques 38 et le convertisseur numérique/analogique 30 pour le contrôle des puits de courant Pᵢ.

De façon caractéristique de l'invention, les sources/puits de courant sont dissociés des pôles de stimulation, avec deux conséquences :
- le nombre M de pôles de stimulation peut être égal au nombre N de sources/puits de courant, ou être différent du nombre de sources/puits de courant. Avantageusement, N > M pour augmenter l'effet d'équilibrage par moyennage des courants appliqués conjointement à un même pôle (comme on le décrira plus bas) ;
- chacune des N sources de courant et chacun des N puits de courant peut être attribué indifféremment, et de façon dynamique (c'est-à-dire modifiable à tout moment), à n'importe lequel des M pôles de stimulation, c'est-à-dire être électriquement connecté à n'importe laquelle des M électrodes.

Les matrices N x M 26 et 36 permettent de relier électriquement n'importe quelle source/puits de courant à n'importe quelle électrode, y compris plusieurs sources/puits à une même électrode. En d'autres termes, chacun des générateurs de courant peut être attribué à volonté, à un instant donné et de manière modifiable, à l'un quelconque des différents pôles de la configuration de stimulation.

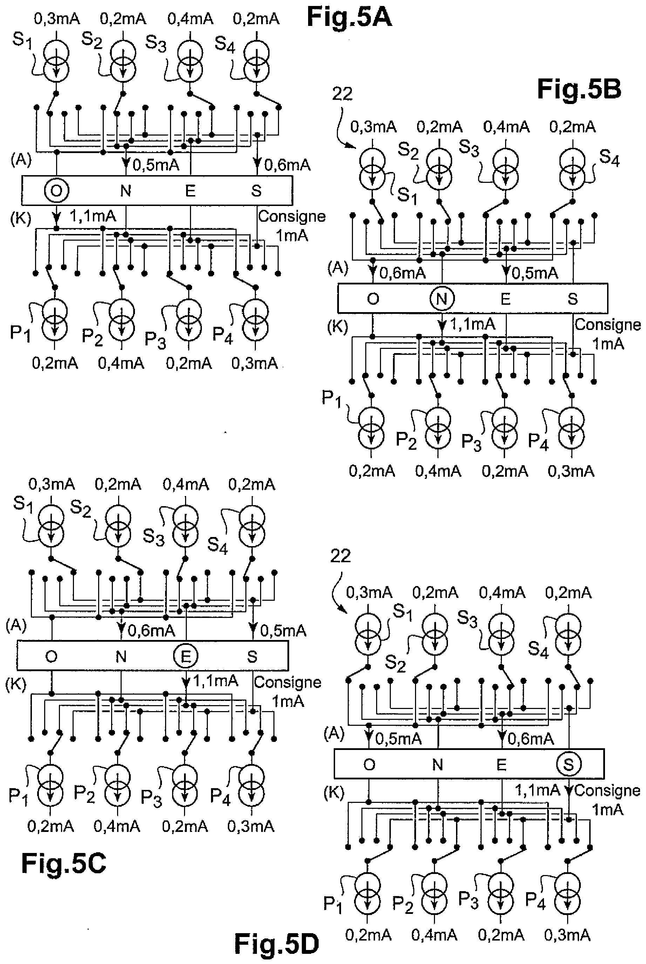

Les Figures 5A à 5D illustrent respectivement la manière d'obtenir, avec une architecture de générateur selon l'invention, quatre configurations polaires différentes de stimulation (étant entendu qu'en augmentant le nombre d'électrodes il est possible de disposer d'un plus grand nombre de configurations de sélectivité possibles).

Ces figures illustrent des configurations de sélectivité respectives Ouest-Nord-Est-Sud, avec l'architecture de générateur de l'invention, à comparer aux configurations homologues respectives Ouest-Nord-Est-Sud des Figures 3A à 3D mettant en œuvre une architecture conventionnelle de générateurs.

On comparera notamment les valeurs des différents courants circulant dans les électrodes actives d'anodes et de cathodes.

Dans le mode de réalisation que l'on va décrire, on prendra par simplicité N = 4 comme nombre de sources/puits de courant, mais ce choix n'est aucunement limitatif.

Dans l'exemple illustré, le pôle définissant côté cathode la configuration de sélectivité est relié conjointement à tous les puits de courant, par exemple dans la Figure 5A qui décrit la configuration de sélectivité Ouest, l'électrode du pôle Ouest est reliée aux quatre puits de courant P₁, P₂, P₃ et P₄.

Côté anode, le pôle précédent (dans le sens trigonométrique), c'est-à-dire le pôle Sud, est connecté conjointement à deux des quatre sources, dans l'exemple illustré les sources S₃ et S₄. Le pôle suivant (dans le sens trigonométrique), c'est-à-dire le pôle Nord, est quant à lui connecté conjointement aux deux autres sources de courant S₁ et S₂.

Les schémas de commutation pour la configuration de sélectivité Nord (Figure 5B), pour la configuration de sélectivité Est (Figure 5C) et pour la configuration de sélectivité Sud (Figure 5D) se déduisent de la configuration de la Figure 5A que l'on vient de décrire par des rotations successives de 90°, en reliant de la même façon l'électrode de cathode à l'ensemble des puits de courant et en reliant chacune des deux électrodes qui la précèdent et qui la suivent à deux des quatre sources de courant, respectivement.

Si l'on observe les courants produits par ces différentes configurations, et la manière dont ils sont répartis, on constate que :
- le courant total injecté est le même (1,1 mA) quelle que soit la configuration de sélectivité. Ce courant injecté total est en fait égal à la somme des courants délivrés par les sources S₁, S₂, S₃ et S₄, soit : 0,3 + 0,2 + 0,4 + 0,2 = 1,1 mA, du fait que dans toutes les configurations les quatre sources sont commutées et injectent leur courant propre dans le nerf ;
- d'autre part, les courants injectés dans les électrodes d'anode sont toujours les mêmes : 0,6 mA pour l'électrode précédant la cathode et 0,5 mA pour l'électrode suivant la cathode (dans le sens trigonométrique).

De la sorte, grâce à cette architecture, le générateur produit un courant injecté dans le nerf qui présente toujours la même valeur (dans l'absolu), dans cet exemple 1,1 mA, même si cette valeur diffère de la valeur de consigne théorique (1 mA). Le courant dans le nerf sera donc toujours le même quelle que soit la configuration choisie, et la seule erreur qui demeure est une erreur systématique, elle n'est pas imprévisible et dépendante de la configuration de stimulation.

On soulignera que l'on a une seule consigne pour l'ensemble des sources et des puits de courant, le courant effectivement délivré étant donc la somme des courants des sources unitaires.

On supprime ainsi le biais que l'on avait avec l'architecture classique lorsque l'on modifiait la configuration de stimulation.

Dès lors, si l'on observe une variation du paramètre physiologique d'une configuration de stimulation à l'autre, cela signifie que cette variation est liée à la configuration différente, et non à la variation du courant total injecté entre des configurations différentes.

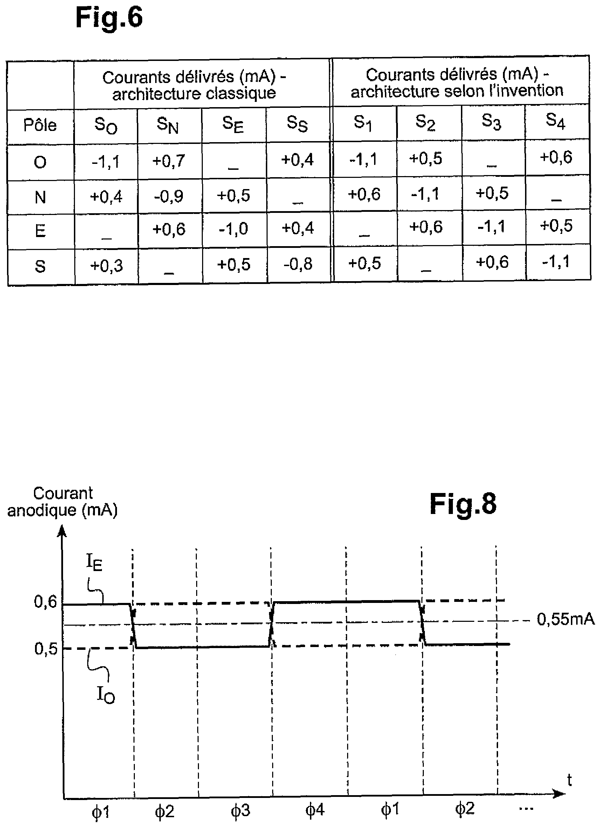

La Figure 6 est un tableau comparatif récapitulant, pour une architecture classique et pour une architecture selon l'invention, les courants entrants ou sortants sur les différentes électrodes actives, en fonction de la configuration de stimulation choisie. Par convention, une valeur négative de courant correspond à un courant sortant du pôle côté cathode, et une valeur positive à un courant entrant dans le pôle côté anode.

On constate que :
- côté cathode, le courant sortant est toujours le même avec l'architecture de l'invention : 1,1 mA au lieu de 0,8 à 1,1 mA avec l'architecture classique ;
- le courant injecté dans le pôle anodique précédant est toujours de 0,5 mA, et
- le courant injecté dans le pôle anodique suivant est toujours de 0,6 mA.

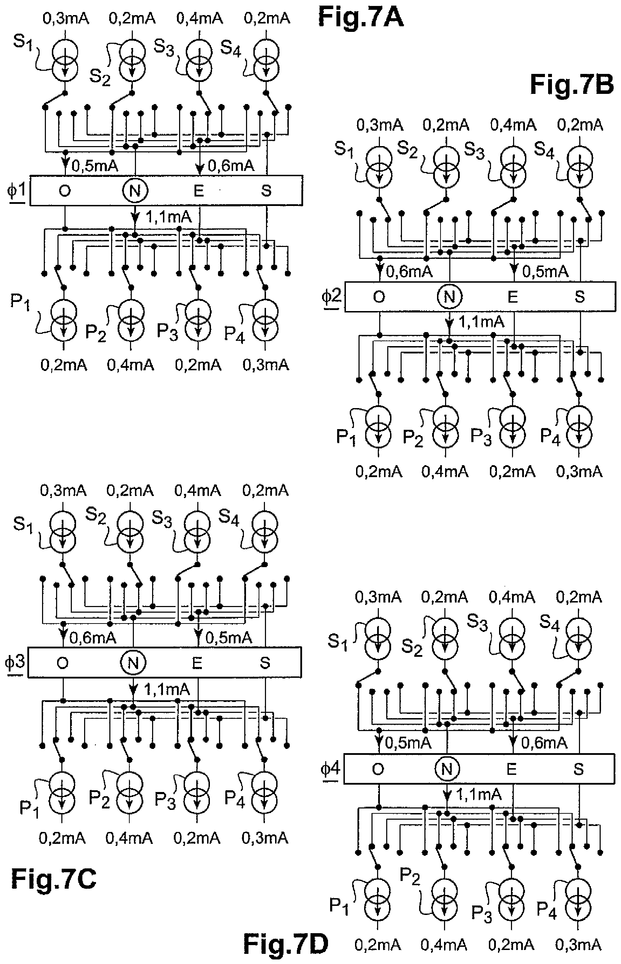

Les Figures 7A à 7D illustrent un perfectionnement de l'invention permettant de corriger les écarts entre les courants injectés côté anode qui, bien qu'ils soient les mêmes d'une configuration à l'autre (0,5 et 0,6 mA dans l'exemple des Figures 5A à 5D), ne sont pas exactement équilibrés (la consigne étant de 0,5 + 0,5 mA).

Le principe de ce perfectionnement consiste, pour une même configuration de stimulation donnée, à piloter de façon dynamique le circuit de commutation 26 en alternance des sources, de façon à moyenner le courant effectivement délivré au niveau de chaque pôle.

On a vu dans l'exemple des Figures 5A à 5D que chacun des deux pôles anodiques était commuté à deux des quatre sources de courant. Dans le cas du perfectionnement considéré, pour une même configuration de stimulation, on opèrera une permutation des sources commutées à ce même pôle d'anode au cours de phases successives de sorte que, au cours de chacune des phases, ce soit toujours deux sources qui soient connectées à ce pôle, mais des sources différentes. La permutation peut être une permutation circulaire, ou tout autre type de permutation. Étant donné que chaque source possède la même consigne, on peut opérer des permutations aléatoires avec l'ensemble des sources d'un côté et l'ensemble des puits de l'autre.

Dans l'exemple des Figures 7A à 7D, on a illustré le pilotage de la configuration de sélectivité Nord selon ce perfectionnement.

Côté cathode, l'électrode Nord reste connectée aux quatre puits de courant conjointement P₁ à P₄.

En revanche, côté anode, on modifie la configuration de commutation des sources au cours de quatre phases Ø1 à Ø4 successives :
- au cours de la phase Ø1, les sources S₁ et S₂ sont connectées à l'électrode Ouest et les sources S₃ et S₄ à l'électrode Est ;
- au cours de la phase Ø2, les sources S₂ et S₃ sont connectées à l'électrode Ouest et les sources S₁ et S₄ sont connectées à l'électrode Est ;
- au cours de la phase Ø3, les sources S₃ et S₄ sont connectées à l'électrode Ouest et les sources S₁ et S₂ sont connectées à l'électrode Est ;
- au cours de la phase Ø4, les sources S₁ et S₄ sont connectées à l'électrode Ouest et les sources S₂ et S₃ sont connectées à l'électrode Est.

Cette commutation en quatre phases successives Ø1 à Ø4 est exécutée pendant toute la durée de la neurostimulation.

La Figure 8 est un chronogramme représentant la succession des différentes phases Ø1 ... Ø4, Ø1, Ø2 ... et les courants anodiques I_{O} et I_{E} injectés respectivement dans les électrodes Ouest et Est.

La technique de commutation dynamique permet de moyenner le courant dans l'électrode Ouest (0,5 mA, puis 0,6 mA, puis 0,5 mA, etc.) et celui dans l'électrode Est (0,6 mA, puis 0,5 mA, puis 0,6 mA, etc.) à une valeur uniforme de 0,55 mA dans chacune des deux électrodes de la configuration polaire de stimulation considérée, ce qui permet de réaliser une symétrie véritable entre les deux électrodes d'anode (Ouest et Est) entourant l'électrode cathodique (Nord) définissant la configuration de sélectivité choisie.

En répétant la même opération sur toutes les configurations polaires, on garantit que le courant moyen (charge électrique totale délivrée) reste constant quelle que soit la configuration polaire.

Pour assurer un bon moyennage, il est souhaitable que les durées des quatre phases Ø1 à Ø4 soient toutes égales. La durée d'une phase élémentaire est, idéalement, la plus réduite possible pour maximiser l'effet de moyennage sur la durée de la neurostimulation si celle-ci est produite sous forme d'une impulsion relativement brève. Concrètement, cette durée dépend des contraintes technologiques de temps de commutation des différents interrupteurs, etc.

## Revendications

1. Un dispositif médical implantable actif de neurostimulation par injection contrôlée de courants électriques simultanément en plusieurs points d'un tissu physiologique, comprenant :
- une sonde de neurostimulation (12) apte à être placée autour, à proximité ou à l'intérieur d'un organe (VN), comportant au moins un agencement d'électrodes sectorielles (E_{O}, E_{N}, E_{E}, E_{S}) aptes à former des pôles de stimulation avec passage d'un courant de neurostimulation entre au moins une anode (A) et au moins une cathode (K) d'une configuration de stimulation prédéterminée ; et
- un générateur (10) d'impulsions électriques de courant, comprenant :
• une pluralité de sources de courant (S_{O}, S_{N}, S_{E}, S_{S} ; S₁, S₂, S₃, S₄) ;
• une pluralité de puits de courant (P_{O}, P_{N}, P_{E}, P_{S} ; P₁, P₂, P₃, P₄) ;
• une première structure de distribution des courants issus desdites sources de courant (16 ; 26), pour le couplage sélectif d'au moins l'une des sources de courant à une électrode, de sorte que cette électrode constitue une électrode active d'anode de ladite configuration de stimulation prédéterminée ;
• une seconde structure de distribution des courants issus desdits puits de courant (20 ; 36) pour le couplage sélectif d'au moins l'un des puits de courant à une autre électrode, de sorte que cette autre électrode constitue une électrode active de cathode de ladite configuration de stimulation prédéterminée,
dans lequel l'agencement d'électrodes (24) comprend M électrodes (O, N, E, S) et le générateur comprend N sources de courant (22) et N puits de courant (32), avec N = M ou N ≠ M, les N sources de courant et les N puits de courant étant définis indépendamment des M électrodes :
- la première structure de distribution (26) est apte à opérer un couplage indifféremment d'au moins l'une des N sources de courant (S₁, S₂, S₃, S₄) à l'une quelconque des M électrodes (O, N, E, S) ;
- la seconde structure de distribution (36) est apte à opérer un couplage indifféremment d'au moins l'un des N puits de courant (P₁, P₂, P₃, P₄) à l'une quelconque des M électrodes (O, N, E, S) ; et
- le générateur comprend en outre des moyens de pilotage (18) de la première et de la seconde structure de distribution, aptes à définir une combinaison de couplages d'une pluralité de sources de courant et/ou d'une pluralité de puits de courant procurant un même courant moyen de neurostimulation pour des configurations de stimulation prédéterminées respectives différentes ; et
dans lequel les moyens de pilotage sont des moyens de pilotage multiphasique, aptes à produire cycliquement une pluralité de combinaisons de couplages pour une même configuration de stimulation, de manière à moyenner le courant de neurostimulation délivré par la pluralité de sources de courant et la pluralité de puits de courant.

2. Le dispositif de la revendication 1, dans lequel ladite combinaison de couplages inclut un couplage de toutes les sources de courant.

3. Le dispositif de la revendication 2, dans lequel une partie des N sources de courant est couplée conjointement sur une première électrode d'anode, et la partie restante des N sources de courant est couplée conjointement sur une seconde électrode d'anode, différente de la première électrode d'anode.

4. Le dispositif de la revendication 1, dans lequel ladite combinaison de couplages inclut un couplage de tous les puits de courant.

5. Le dispositif de la revendication 4, dans lequel une partie des N puits de courant est couplée conjointement sur une première électrode de cathode, et la partie restante des N puits de courant est couplée conjointement sur une seconde électrode de cathode, différente de la première électrode de cathode.

6. Le dispositif de la revendication 4, dans lequel les N puits de courant sont couplés conjointement sur une unique électrode de cathode.

7. Le dispositif de la revendication 1, dans lequel les moyens de pilotage comprennent des moyens aptes à définir une combinaison de couplages de chacune des sources de courant et de chacun des puits de courant successivement sur chacune des électrodes de la configuration de stimulation prédéterminée.

8. Le dispositif de la revendication 1, dans lequel ladite pluralité de combinaisons de couplages produites cycliquement pour une même configuration de stimulation comprennent une permutation circulaire des N sources de courant.

9. Le dispositif de la revendication 1, dans lequel ladite pluralité de combinaisons de couplages produites cycliquement pour une même configuration de stimulation comprennent une permutation aléatoire des N sources de courant.

10. Le dispositif de la revendication 1, dans lequel les moyens de pilotage sont des moyens aptes à produire cycliquement ladite pluralité de combinaisons de couplages au cours de phases successives de durées respectives égales.

## Patentansprüche

1. Aktive implantierbare medizinische Vorrichtung zur Neurostimulation durch gleichzeitiges, kontrolliertes Einspeisen elektrischer Ströme an mehreren Stellen eines physiologischen Gewebes, mit:
- einer Neurostimulationssonde (12), die um ein Organ, in der Nähe oder im Inneren eines Organs (VN) angeordnet werden kann, mit mindestens einer Anordnung von sektoralen Elektroden (E_{O}, E_{N}, E_{E}, E_{S}), die Stimulationspole bilden können, wobei ein Neurostimulationsstrom zwischen mindestens einer Anode (A) und mindestens einer Kathode (K) mit einer vorbestimmten Stimulationskonfiguration hindurchfließt; und
- einem Generator (10) für elektrische Stromimpulse mit:
• einer Vielzahl von Stromquellen (S_{O}, S_{N}, S_{E}, S_{S} ; S₁, S₂, S₃, S₄);
• einer Vielzahl von Stromsenken (P_{O}, P_{N}, P_{E}, P_{S} ; P₁, P₂, P₃, P₄);
• einer ersten Struktur zur Verteilung des von den Stromquellen (16; 26) stammenden Stromes zum selektiven Koppeln von mindestens einer der Stromquellen an eine Elektrode, so dass diese Elektrode eine aktive Anodenelektrode mit der vorbestimmten Stimulationskonfiguration bildet;
• einer zweiten Struktur zur Verteilung des von den Stromsenken (20; 36) stammenden Stromes zum selektiven Koppeln von mindestens einer der Stromsenken an eine andere Elektrode, so dass diese andere Elektrode eine aktive Kathodenelektrode mit der vorbestimmten Stimulationskonfiguration bildet,
bei der die Elektrodenanordnung (24) M Elektroden (O, N, E, S) umfasst und der Generator N Stromquellen (22) und N Stromsenken (32) umfasst, mit N = M oder N ≠ M, wobei die N Stromquellen und die N Stromsenken unabhängig von den M Elektroden definiert sind:
- die erste Verteilungsstruktur (26) ist in der Lage, mindestens eine der N Stromquellen (S₁, S₂, S₃, S₄) beliebig mit einer der M Elektroden (O, N, E, S) zu koppeln;
- die zweite Verteilungsstruktur (36) ist in der Lage, mindestens eine der N Stromsenken (P₁, P₂, P₃, P₄) beliebig mit einer der M Elektroden (O, N, E, S) zu koppeln; und
- der Generator umfasst ferner Mittel (18) zum Steuern der ersten und zweiten Verteilungsstruktur, die in der Lage sind, eine Kombination von Kopplungen aus einer Vielzahl von Stromquellen und/oder einer Vielzahl von Stromsenken zu definieren, die den gleichen mittleren Neurostimulationsstrom für verschiedene, jeweils vorbestimmte Stimulationskonfigurationen liefern; und
wobei die Steuermittel mehrphasige Steuermittel sind, die in der Lage sind, zyklisch eine Vielzahl von Kopplungskombinationen für die gleiche Stimulationskonfiguration zu erzeugen, um den von der Vielzahl von Stromquellen und der Vielzahl von Stromsenken gelieferten Neurostimulationsstrom zu mitteln.

2. Vorrichtung nach Anspruch 1, bei der die Kombination von Kopplungen eine Kopplung aller Stromquellen beinhaltet.

3. Vorrichtung nach Anspruch 2, bei der ein Teil der N Stromquellen an einer ersten Anodenelektrode zusammengekoppelt ist und der verbleibende Teil der N Stromquellen an einer zweiten Anodenelektrode zusammengekoppelt ist, die sich von der ersten Anodenelektrode unterscheidet.

4. Vorrichtung nach Anspruch 1, bei der die Kombination von Kopplungen eine Kopplung aller Stromsenken beinhaltet.

5. Vorrichtung nach Anspruch 4, bei der ein Teil der N Stromsenken an einer ersten Kathodenelektrode zusammengekoppelt ist und der verbleibende Teil der N Stromsenken an einer zweiten Kathodenelektrode zusammengekoppelt ist, die sich von der ersten Kathodenelektrode unterscheidet.

6. Vorrichtung nach Anspruch 4, bei der die N Stromsenken an einer einzigen Kathodenelektrode zusammengekoppelt sind.

7. Vorrichtung nach Anspruch 1, bei der die Steuermittel Mittel umfassen, die in der Lage sind, eine Kombination von Kopplungen jeder der Stromquellen und jeder der Stromsenken nacheinander an jeder der Elektroden mit der vorbestimmten Stimulationskonfiguration zu definieren.

8. Vorrichtung nach Anspruch 1, bei der die Vielzahl von Kombinationen von zyklisch erzeugten Kopplungen für ein und dieselbe Stimulationskonfiguration eine kreisförmige Permutation der N Stromquellen beinhaltet.

9. Vorrichtung nach Anspruch 1, bei der die Vielzahl von Kombinationen von zyklisch erzeugten Kopplungen für ein und dieselbe Stimulationskonfiguration eine zufällige Permutation der N Stromquellen beinhaltet.

10. Vorrichtung nach Anspruch 1, bei der die Steuermittel Mittel sind, die in der Lage sind, die Vielzahl von Kopplungskombinationen während aufeinander folgender Phasen von jeweils gleicher Dauer zyklisch zu erzeugen.

## Claims

1. An active implantable medical device for neurostimulation by controlled injection of electric currents simultaneously at a plurality of points of a physiological tissue, said active implantable medical device comprising:
- a neurostimulation lead (12) suitable for being placed around, in the vicinity of, or inside an organ (VN), which neurostimulation lead includes at least one arrangement of sectoral electrodes (E_{O}, E_{N}, E_{E}, E_{S}) that are suitable for forming stimulation poles with a neurostimulation current flowing between at least one anode (A) and at least one cathode (K) of a predetermined stimulation configuration; and
- a generator (10) of electric current pulses, which generator comprises:
• a plurality of current sources (S_{O}, S_{N}, S_{E}, S_{S}; S₁, S₂, S₃, S₄);
• a plurality of current sinks (P_{O}, P_{N}, P_{E}, P_{S} ; P₁, P₂, P₃, P₄);
• a first distribution structure for distributing currents coming from said current sources (16; 26), for selectively coupling at least one of the current sources to an electrode, so that said electrode constitutes an anode active electrode of said predetermined stimulation configuration; and
• a second distribution structure for distributing currents coming from said current sinks (20; 36) for selectively coupling at least one of the current sinks to another electrode so that said other electrode constitutes a cathode active electrode of said predetermined stimulation configuration;
wherein the arrangement of electrodes (24) comprises M electrodes (O, N, E, S) and the generator comprises N current sources (22) and N current sinks (32), where N = M or N ≠ M, the N current sources and the N current sinks being defined independently from the M electrodes:
- the first distribution structure (26) is suitable for coupling indifferently at least one of the N current sources (S₁, S₂, S₃, S₄) to any one of the M electrodes (O, N, E, S);
- the second distribution structure (36) is suitable for coupling indifferently at least one of the N current sinks (P₁, P₂, P₃, P₄) to any one of the M electrodes (O, N, E, S); and
- the generator further comprises control means (18) for controlling the first and second distribution structures, which control means are suitable for defining a combination of couplings of a plurality of current sources and/or of a plurality of current sinks procuring the same average neurostimulation current for different respective predetermined stimulation configurations; and
wherein the control means are multi-phase control means suitable for cyclically producing a plurality of combinations of couplings for the same stimulation configuration, in such a manner as to average the neurostimulation current delivered by the plurality of current sources and the plurality of current sinks.

2. The device of claim 1, wherein said combination of couplings includes a coupling of all of the current sources.

3. The device of claim 2, wherein some of the N current sources are coupled jointly to a first anode electrode, and the remaining ones of the N current sources are coupled jointly to a second anode electrode that is different from the first anode electrode.

4. The device of claim 1, wherein said combination of couplings includes a coupling of all of the current sinks.

5. The device of claim 4, wherein some of the N current sinks are coupled jointly to a first cathode electrode, and the remaining ones of the N current sinks are coupled jointly to a second cathode electrode that is different from the first cathode electrode.

6. The device of claim 4, wherein the N current sinks are coupled jointly to a single cathode electrode.

7. The device of claim 1, wherein the control means comprise means suitable for defining a combination of couplings of each of the current sources and of each of the current sinks successively to each of the electrodes of the predetermined stimulation configuration.

8. The device of claim 1, wherein said plurality of combinations of couplings produced cyclically for the same stimulation configuration comprise a circular permutation of the N current sources.

9. The device of claim 1, wherein said plurality of combinations of couplings produced cyclically for the same stimulation configuration comprise a random permutation of the N current sources.

10. The device of claim 1, wherein the control means are means suitable for cyclically producing said plurality of combinations of couplings during successive phases of respective equal durations.
